# EUROPEAN PATENT APPLICATION

(11) **EP 3 636 266 A1**
(43) Date of publication of application: **15.04.2020**
(21) Application number: 18814375.4
(22) Date of filing: 05.06.2018
(51) Int. Cl.: A61K 31/7032, A61K 8/60, A61P 17/02, A61P 29/00

(54) **AGENT FOR INHIBITING SKIN TROUBLE AND COMPOSITION FOR INHIBITING SKIN TROUBLE**

(30) Priority: 05.06.2017 JP 2017111078
(71) Applicant: Showa Denko K.K., Tokyo 105-8518 (JP)
(72) Inventor: NAKAGAMI Yuko, Tokyo 105-8518 (JP); MISHINA Natsuno, Tokyo 105-8518 (JP); KATO Eiko, Tokyo 105-8518 (JP)
(74) Representative: Strehl Schübel-Hopf & Partner
(86) International application number: PCT/JP2018/021529
(87) International publication number: WO 2018/225718

(57) **Abstract**

An agent for inhibiting skin trouble caused by fine particulate matter includes, as an active ingredient, an inositol derivative in which a sugar binds to inositol. In addition, a composition for inhibiting skin trouble caused by fine particulate matter includes the above-mentioned agent for inhibiting skin trouble, and a pharmaceutically acceptable carrier.

## Description

### [Technical Field]

The present invention relates to an agent for inhibiting skin trouble, and a composition for inhibiting skin trouble.

Priority is claimed on Japanese Patent Application No. 2017-111078, filed June 5, 2017, the content of which is incorporated herein by reference.

### [Background Art]

In recent years, atmospheric pollution due to fine particulate matter has become severe, and fine particulate matter is known to adversely affect not only respiratory diseases and cardiovascular diseases but also skin. For this reason, external preparations for skin that reduce damage to skin caused by the fine particulate matter have been studied.

Patent Literature 1 discloses a composition for protecting skin from atmospheric contaminants, acidic liquids, and ultraviolet rays, and this composition is characterized by containing magnesium aluminometasilicate and an ultraviolet protective agent.

Patent Literature 2 discloses an external preparation for skin for preventing heavy-metal contamination of skin and for washing skin contaminated by heavy metals, and this preparation is characterized by containing an inorganic powder such as pulverized zeolite.

Patent Literature 3 discloses a pollutant inhibitor that prevents atmospheric contaminants from penetrating into skin, and this inhibitor is characterized by containing an α-gel structure.

The external preparations for skin disclosed in Patent Literature 1 to Patent Literature 3 are intended to protect skin from irritant substances such as atmospheric contaminants, and are preparations that physically protect the skin.

Meanwhile, Patent Literature 4 discloses an inhibitor against skin inflammation caused by atmospheric aerosol particles, and this inhibitor is characterized by containing an extract of the genus *Hippophae* belonging to the family *Elaeagnaceae* as an active ingredient. In addition, Non-Patent Literature 1 discloses that inositol reduces cell cytotoxicity occurring when epidermal cells are treated with a surfactant.

### [Citation List]

### [Patent Literature]

[Patent Literature 1]
   PCT International Publication No. WO2014/136993
[Patent Literature 2]
   Japanese Unexamined Patent Application, First Publication No. 2013-107830
[Patent Literature 3]
   Japanese Unexamined Patent Application, First Publication No. 2016-88866
[Patent Literature 4]
   Japanese Unexamined Patent Application, First Publication No. 2016-216366

### [Non-Patent Literature]

[Non-Patent Literature 1]
Oils and fats, Vol. 65, No. 8, p. 48-54, 2012.

### [Summary of Invention]

### [Technical Problem]

The external preparations for skin disclosed in Patent Literature 1 to Patent Literature 3 physically protect skin, but do not inhibit skin trouble of skin damaged due to contact with fine particulate matter. In addition, the inhibitor against skin inflammation disclosed in Patent Literature 4 shows an effect of inhibiting the production of inflammation-inducing substances, but the inhibiting effect thereof is insufficient. The inositol described in Non-Patent Literature 1 has been tested for cell cytotoxicity occurring due to a surfactant, but does not show the effect of inhibiting the production of inflammation-inducing substances.

Furthermore, these external preparations for skin of the related art are not preparations that can inhibit skin trouble by restoring skin damaged by fine particulate matter.

Accordingly, an object of the present invention is to provide an agent for inhibiting skin trouble and a composition for inhibiting skin trouble which effectively inhibit skin trouble caused by fine particulate matter through effects of inhibiting the production of inflammation-inducing substances and activating cells.

### [Solution to Problem]

The present invention includes the following aspects.
(1) An agent for inhibiting skin trouble caused by fine particulate matter, the agent including, as an active ingredient, an inositol derivative in which a sugar binds to inositol.
(2) The agent for inhibiting skin trouble according to (1), in which the sugar is a monosaccharide or an oligosaccharide.
(3) The agent for inhibiting skin trouble according to (2), in which the monosaccharide is glucose.
(4) The agent for inhibiting skin trouble according to (2), in which the oligosaccharide is an oligosaccharide containing glucose as a structural unit.
(5) The agent for inhibiting skin trouble according to any one of (1) to (4), in which the inositol is myo-inositol.
(6) The agent for inhibiting skin trouble according to any one of (1) to (5), which inhibits the production of an inflammation-inducing substance.
(7) The agent for inhibiting skin trouble according to any one of (1) to (6), which promotes cell activation.
(8) A composition for inhibiting skin trouble caused by fine particulate matter, the composition including the agent for inhibiting skin trouble according to any one of (1) to (7), and a pharmaceutically acceptable carrier.
(9) The composition for inhibiting skin trouble according to (8), in which the content of the inositol derivative is 0.01% to 50% by mass.
(10) The composition for inhibiting skin trouble according to (8) or (9), which is an external preparation for skin.
(11) The composition for inhibiting skin trouble according to any one of (8) to (10), which is a cosmetic preparation.

### [Advantageous Effects of Invention]

According to the present invention, it is possible to provide an agent for inhibiting skin trouble and a composition for inhibiting skin trouble which effectively inhibit skin trouble caused by fine particulate matter.

### [Description of Embodiments]

### [Agent for inhibiting skin trouble caused by fine particulate matter]

In one embodiment, the present invention provides an agent for inhibiting skin trouble caused by fine particulate matter, the agent including, as an active ingredient, an inositol derivative in which a sugar binds to inositol.

The fine particulate matter refers to a particle of 2.5 µm or less suspended in the atmosphere, and is also called PM2.5. More specifically, it refers to particles that are particulate matter suspended in the atmosphere and collected after removing particles having a larger particle diameter by using a sizing apparatus that can separate, at a ratio of 50%, particles which have a particle diameter of 2.5 µm.

In the present specification, the phrase "skin trouble caused by fine particulate matter" means skin disorders induced due to contact of the skin with the fine particulate matter. Examples of skin trouble caused by the fine particulate matter include inflammation, but examples are not limited thereto.

As will be described later in Examples, the agent for inhibiting skin trouble of the present embodiment acts to restore cell proliferative activity of human epidermal keratinocytes in which cell proliferative activity has been reduced due to damage of the cells by the fine particulate matter. In addition, the agent for inhibiting skin trouble of the present embodiment acts to inhibit the production of an inflammation-inducing substance induced by the fine particulate matter. According to these actions, the agent for inhibiting skin trouble of the present embodiment can effectively inhibit skin trouble caused by the fine particulate matter.

In addition, since the agent for inhibiting skin trouble of the present embodiment has the above-described actions, it can be said to promote cell activation of skin cells in which cell proliferative activity has been reduced due to the fine particulate matter. Furthermore, the agent for inhibiting skin trouble of the present embodiment can also be said to be a cell-activating agent for skin cells in which cell proliferative activity has been reduced due to the fine particulate matter. Moreover, the agent for inhibiting skin trouble of the present embodiment can also be said to inhibit the production of an inflammation-inducing substance in the skin cells which is caused by the fine particulate matter. Moreover, the agent for inhibiting skin trouble of the present embodiment can also be said to be an inhibitor for the production of an inflammation-inducing substance in the skin cells which is caused by the fine particulate matter. Moreover, the agent for inhibiting skin trouble of the present embodiment can also be said to be an anti-inflammatory agent for inflammation induced by the fine particulate matter.

In the present specification, the term "cell activation" means that cell proliferative activity of cells is improved. In other words, the cell-activating agent is a drug that improves cell proliferative activity of cells. The agent for inhibiting skin trouble of the present embodiment effectively promotes cell activation of skin cells damaged by the fine particulate matter. Such cell activation includes restoring of cell proliferative activity in skin cells in which cell proliferative activity has been reduced due to damage of the cells by the fine particulate matter. In addition, the cell activation includes inhibition of a reduction in cell proliferative activity caused by the fine particulate matter.

In the present specification, the "inflammation-inducing substance" refers to an endogenous substance that induces an inflammatory response. The inflammation-inducing substance is not particularly limited as long as it is a substance that is produced in vivo and induces an inflammatory response, and examples thereof include prostaglandins, histamines, kinins, leukotrienes, TNF-α, and the like. In particular, the agent for inhibiting skin trouble of the present embodiment can effectively inhibit the production of an inflammation-inducing substance produced by skin cells upon being damaged by the fine particulate matter. Examples of such inflammation-inducing substances include prostaglandins, and more specific examples thereof include prostaglandin E2.

### (Inositol derivative)

The agent for inhibiting skin trouble of the present embodiment contains, as an active ingredient, an inositol derivative in which a sugar binds to inositol.

Inositol is a cyclic hexahydric alcohol represented by C₆H₆(OH)₆. There are nine stereoisomeric forms of inositol: cis-inositol, epi-inositol, allo-inositol, myo-inositol, muco-inositol, neo-inositol, chiro-inositol (D-chiro- and L-chiro-), and scyllo-inositol.

In the agent for inhibiting skin trouble of the present embodiment, the inositol constituting the inositol derivatives is preferably myo-inositol, which is the only one that is physiologically active among the above-mentioned isomeric forms. Inositol can be synthesized by a method of extraction from rice bran, a chemical synthesis method, a fermentation method, or the like.

In the agent for inhibiting skin trouble of the present embodiment, the inositol derivatives are compounds in which a sugar binds to a hydroxyl group of inositol. The sugar may bind to any one of six hydroxyl groups present in an inositol molecule, or may bind to any two or more thereof.

A sugar that binds to inositol may be a monosaccharide or an oligosaccharide. For example, one or more monosaccharides may bind to one inositol molecule, one or more oligosaccharides may bind to one inositol molecule, or one or more monosaccharides and one or more oligosaccharides may bind to one inositol molecule. In the inositol derivatives, a total number of monosaccharides or oligosaccharides binding to one inositol molecule is 1 or more, may be 2 or more for example, may be 3 or more for example, and may be 4 or more for example in terms of monosaccharide units.

In the present specification, a monosaccharide refers to a sugar group that cannot be further hydrolyzed, and refers to a compound that is a constituent element when forming a polysaccharide. A monosaccharide can also be said to be the smallest structural unit of a sugar group. In the present specification, the term "monosaccharide unit" refers to a chemical structure corresponding to a monosaccharide. A "monosaccharide unit" can also be said to be a chemical structure derived from a monosaccharide. For example, a disaccharide is converted into two monosaccharide units, and a trisaccharide is converted into three monosaccharide units. More specifically, for example, mannitol, sorbitol, xylitol, erythritol, pentaerythritol, glucose, fructose, xylose, or the like is converted into one monosaccharide unit.

In addition, maltitol, sucrose, lactose, maltose, trehalose, or the like is converted into two monosaccharide units. Furthermore, for example, α-cyclodextrin is converted into six monosaccharide units; β-cyclodextrin is converted into seven monosaccharide units; and γ-cyclodextrin is converted into eight monosaccharide units.

The inositol derivatives may be a mixture of inositol derivatives to which different numbers of sugars bind in terms of monosaccharide units. For example, the inositol derivatives may be a mixture of an inositol derivative in which one saccharide in terms of monosaccharide units binds to one inositol molecule, an inositol derivative in which two saccharides in terms of monosaccharide units bind to one inositol molecule, an inositol derivative in which three saccharides in terms of monosaccharide units bind to one inositol molecule, an inositol derivative in which four saccharides in terms of monosaccharide units bind to one inositol molecule, and an inositol derivative in which five or more saccharides in terms of monosaccharide units bind to one inositol molecule. For example, the inositol derivatives may be inositol derivatives containing 10% to 100% by mass of inositol derivatives in which two or more saccharides in terms of monosaccharide units bind to one inositol molecule with respect to a total mass (100%) of the inositol derivatives. A proportion of the inositol derivative in which two or more saccharides in terms of monosaccharide units bind to one inositol molecule in the total mass (100%) of the inositol derivatives is, for example, 20% by mass or more, 30% by mass or more, 40% by mass or more, 50% by mass or more, 60% by mass or more, 70% by mass or more, or 80% by mass or more.

A sugar constituting the inositol derivatives is not particularly limited, and examples thereof include mannitol, sorbitol, xylitol, maltitol, erythritol, pentaerythritol, glucose, sucrose, fructose, lactose, maltose, xylose, trehalose, α-cyclodextrin, β-cyclodextrin, γ-cyclodextrin, and the like.

The sugar constituting the inositol derivatives may be glucose, or an oligosaccharide containing glucose as a structural unit. The oligosaccharide may contain only glucose as a structural unit. Alternatively, the oligosaccharide may contain at least one glucose molecule, and a sugar other than glucose as structural units. A molecular weight of the oligosaccharide may be, for example, about 300 to 3000. More specific examples of oligosaccharides include disaccharides such as sucrose, lactose, maltose, trehalose, and cellobiose; trisaccharides such as raffinose, melezitose, and maltotriose; tetrasaccharides such as stachyose; and the like.

The inositol derivatives may be a mixture of an inositol derivative in which the sugar is a monosaccharide, and an inositol derivative in which the sugar is an oligosaccharide. The inositol derivatives may be a mixture of inositol derivatives to which different types of sugars bind.

From the viewpoint of easily obtaining highly purified inositol derivatives, it is preferable to use β-cyclodextrin which is industrially inexpensive and can be stably supplied as a raw material for the inositol derivatives. In this case, the sugar constituting the inositol derivatives contains glucose as a structural unit. Meanwhile, when cheaper starch or the like is used as a raw material for the inositol derivatives, various sugars are transferred to various places during synthesis of inositol derivatives, and thus a degree of purification of inositol derivatives to be obtained tends to become unstable.

In addition, the inositol derivatives may be in a form of a pharmaceutically acceptable salt. In the present specification, the term "pharmaceutically acceptable salt" refers to a salt form that does not inhibit the effect of inhibiting skin trouble caused by the fine particulate matter, which is the effect exhibited by the inositol derivatives. The pharmaceutically acceptable salts of the inositol derivatives are not particularly limited, and examples thereof include salts with alkali metals (sodium, potassium, and the like); salts with alkaline earth metals (magnesium, calcium, and the like); salts with organic bases (pyridine, triethylamine, and the like); salts with amines; salts with organic acids (acetic acid, formic acid, propionic acid, fumaric acid, maleic acid, succinic acid, tartaric acid, citric acid, malic acid, oxalic acid, benzoic acid, methanesulfonic acid, and the like); salts with inorganic acids (hydrochloric acid, phosphoric acid, hydrobromic acid, sulfuric acid, nitric acid, and the like); and the like.

In addition, the inositol derivatives may be in a form of a solvate. Furthermore, the inositol derivative may be in a form of a solvate of salts of the inositol derivative. The solvate is not particularly limited, but examples thereof include hydrates, ethanol solvates, and the like.

### (Method for synthesizing inositol derivatives)

A method for synthesizing the inositol derivatives is not particularly limited, and the inositol derivatives can be appropriately synthesized by a known method of the related art. For example, the inositol derivatives may be synthesized by reacting inositol with cyclodextrin, which is one kind of oligosaccharide, in the presence of cyclodextrin glucanotransferase (refer to, for example, Japanese Unexamined Patent Application, First Publication No. S63-196596). Alternatively, the inositol derivatives may be synthesized by a method for obtaining glucosyl derivatives using glucosyl phosphite as a sugar donor (refer to, for example, Japanese Unexamined Patent Application, First Publication No. H6-298783).

The agent for inhibiting skin trouble of the present embodiment may contain, as the inositol derivatives, one of compounds selected from the group consisting of the above-mentioned inositol derivatives, salts of the inositol derivatives, and solvates thereof, or may contain two or more kinds thereof in combination.

The agent for inhibiting skin trouble of the present embodiment can itself be administered to patients to be used for the purpose of inhibiting skin trouble caused by the fine particulate matter. In addition, the agent for inhibiting skin trouble of the present embodiment can be blended into pharmaceuticals or cosmetic preparations to be used for the purpose of imparting a function of inhibiting skin trouble caused by the fine particulate matter. Furthermore, it can be blended into a composition for inhibiting skin trouble to be described later and used.

The agent for inhibiting skin trouble of the present embodiment may be administered to patients before skin comes into contact with the fine particulate matter to be used for preventing skin trouble such as inflammation caused by the fine particulate matter.

In addition, the agent for inhibiting skin trouble of the present embodiment may be administered to patients who have developed skin trouble due to contact of skin with the fine particulate matter to be used for treating skin trouble such as inflammation caused by the fine particulate matter.

The agent for inhibiting skin trouble of the present embodiment can be administered to patients in the same manner as the composition for inhibiting skin trouble to be described later, and it is preferably administered transdermally.

### [Composition for inhibiting skin trouble caused by fine particulate matter]

In one embodiment, the present invention provides a composition for inhibiting skin trouble caused by fine particulate matter, the composition including the above-mentioned agent for inhibiting skin trouble, and a pharmaceutically acceptable carrier.

The composition for inhibiting skin trouble of the present embodiment can be manufactured by mixing the above-described agent for inhibiting skin trouble, the pharmaceutically acceptable carrier, and optionally, other ingredients, and formulating them according to a general method (for example, a method described in the Japanese Pharmacopoeia).

In the present specification, the term "pharmaceutically acceptable carrier" refers to a carrier that does not inhibit physiological activity of an active ingredient and does not exhibit substantial toxicity with respect to its administration subject. The phrase "not exhibiting substantial toxicity" means that the component is not toxic to an administration subject if it is used at a general dosage. The pharmaceutically acceptable carrier is not particularly limited, and examples thereof include excipients, binders, disintegrants, lubricants, emulsifiers, stabilizers, diluents, solvents for injections, oily bases, moisturizers, touch sensation improvers, surfactants, polymers, thickening/gelling agents, solvents, propellants, antioxidants, reducing agents, oxidizing agents, chelating agents, acids, alkali, powders, inorganic salts, water, metal-containing compounds, unsaturated monomers, polyhydric alcohols, polymer additives, adjuvants, wetting agents, thickeners, tackifiers, oily raw materials, liquid matrices, fat-soluble substances, polymer carboxylate salts, and the like.

Specific examples of these components include those described in PCT International Publication No. WO2016/076310. The pharmaceutically acceptable carrier may be used alone or in combination of two or more kinds thereof.

In addition, the other components are not particularly limited, but examples thereof include preservatives, antibacterial agents, ultraviolet absorbents, whitening agents, vitamins and derivatives thereof, antiphlogistics, anti-inflammatory agents, hair growth agents, blood circulation promoters, stimulants, hormones, anti-wrinkle agents, anti-aging agents, tightening agents, cooling sensation agents, warming sensation agents, wound healing promoters, irritation-relieving agents, analgesics, cell activators, extracts of plants, animals, and microorganisms, antipruritic agents, exfoliating/dissolving agents, antiperspirants, refreshing agents, astringents, enzymes, nucleic acids, fragrances, colorants, coloring agents, dyes, pigments, anti-inflammatory analgesics, antifungals, antihistamines, hypnotic sedatives, tranquilizers, antihypertensives, antihypertensive diuretics, antibiotics, anesthetics, antibacterial substances, antiepileptic agents, coronary vasodilators, herbal medicines, antipruritic agents, keratin softening and exfoliating agents, ultraviolet blockers, antiseptic disinfectants, antioxidant substances, pH adjusters, additives, metal soaps, and the like. Specific examples of these components include those described in PCT International Publication No. WO2016/076310. The other components may be used alone or in combination of two or more kinds thereof.

The composition for inhibiting skin trouble of the present embodiment may be a pharmaceutical composition or a cosmetic preparation.

### (Pharmaceutical composition)

In one embodiment, the present invention provides a pharmaceutical composition for preventing or treating skin trouble caused by fine particulate matter, the composition including the above-mentioned agent for inhibiting skin trouble, and a pharmaceutically acceptable carrier.

In the pharmaceutical composition of the present embodiment, the pharmaceutically acceptable carrier is not particularly limited, and carriers generally used for pharmaceuticals can be used in addition to the carriers exemplified above. For example, it is possible to use general raw materials described in the Japanese Pharmacopoeia, the Japanese Pharmaceutical Codex, the Japanese Pharmaceutical Excipients 2013 (Yakuji Nippo, Ltd., 2013), the Japanese Pharmaceutical Excipients Directory 2016 (Edited by International Pharmaceutical Excipients Council Japan, Yakuji Nippo, Ltd., 2016), Handbook of Pharmaceutical Excipients, 7th edition (Pharmaceutical Press, 2012), and the like. The pharmaceutically acceptable carrier may be used alone or in combination of two or more kinds thereof.

In addition to the agent for inhibiting skin trouble and the pharmaceutically acceptable carrier, the pharmaceutical composition of the present embodiment may contain other components. The other components are not particularly limited, and general pharmaceutical additives can be used. In addition, active ingredients other than the above-described agent for inhibiting skin trouble can be used as the other components. As pharmaceutical additives and active ingredients as the other ingredients, in addition to those exemplified above, it is possible to use general raw materials described in, for example, the Japanese Pharmacopoeia, the Japanese Pharmaceutical Codex, the Japanese Pharmaceutical Excipients 2013 (Yakuji Nippo, Ltd., 2013), the Japanese Pharmaceutical Excipients Directory 2016 (Edited by International Pharmaceutical Excipients Council Japan, Yakuji Nippo, Ltd., 2016), Handbook of Pharmaceutical Excipients, 7th edition (Pharmaceutical Press, 2012), and the like. The other components may be used alone or in combination of two or more kinds thereof.

A dosage form of the pharmaceutical composition of the present embodiment is not particularly limited, and it can be a dosage form generally used for pharmaceutical preparations. Examples thereof include orally administered dosage forms such as tablets, coated tablets, pills, powders, granules, capsules, solutions, suspensions, and emulsions; parenterally administered dosage forms such as injections, suppositories, and external preparations for the skin; and the like. The pharmaceutical composition in these dosage forms can be formulated according to a general method (for example, a method described in the Japanese Pharmacopoeia).

As the pharmaceutical composition of the present embodiment, an external preparation for the skin is preferable. More specific examples of external preparations for skin include dosage forms such as creams, lotions, packs, foams, skin cleansers, extracts, plasters, ointments, spirits, suspensions, tinctures, tapes, poultices, liniments, external aerosols, sprays, and gels.

The pharmaceutical composition of the present embodiment can contain a therapeutically effective amount of the above-described agent for inhibiting skin trouble. The term "therapeutically effective amount" refers to an amount of a drug effective for treating or preventing diseases of patients. The therapeutically effective amount may vary depending on a disease state, age, sex, body weight, and the like of an administration subject. In the pharmaceutical composition of the present embodiment, a therapeutically effective amount of the above-described agent for inhibiting skin trouble may be an amount at which the inositol derivatives can inhibit skin trouble (for example, inflammation) caused by the fine particulate matter. For example, a therapeutically effective amount of the above-described agent for inhibiting skin trouble in the pharmaceutical composition of the present embodiment may be 0.01% to 50% by mass as the content of the inositol derivatives in the pharmaceutical composition, and it may be 0.01 % to 30% by mass for example, it may be 0.01% to 20% by mass for example, it may be 0.1% to 10% by mass for example, it may be 0.1% to 5% by mass for example, it may be 0.1% to 3% by mass for example, it may be 0.3% to 2% by mass for example, and it may be 0.6% to 1.5% by mass for example.

Regarding the content of the above-described inositol derivatives in the pharmaceutical composition, in a case of using one kind of the inositol derivatives, the content refers to the content of this compound, and in a case of using two or more kinds of the inositol derivatives in combination, the content refers to the total content of these compounds.

A method for administering the pharmaceutical composition of the present embodiment is not particularly limited, and the pharmaceutical composition can be administered by a method generally used as a method for administering pharmaceuticals. For example, it may be administered orally as tablets, coated tablets, pills, powders, granules, capsules, solutions, suspensions, emulsions, and the like; it may be administered intravenously, intraarterially, intramuscularly, intradermally, subcutaneously, intraperitoneally, and the like as injections, as infusion preparations, and the like alone, or as a mixture with common infusions such as a glucose solution and a Ringer's solution; it may be administered rectally as suppositories; or it may be administered to skin as external preparations for the skin. In a preferable aspect, the pharmaceutical composition of the present embodiment is applied, affixed, or sprayed to an affected area as external preparations for the skin.

A dosage of the pharmaceutical composition of the present embodiment can be a therapeutically effective amount. The therapeutically effective amount may be appropriately determined according to symptoms, body weight, age, sex, and the like of a patient, a dosage form of the pharmaceutical composition, an administration method, and the like. For example, in the case of oral administration, a dosage of the pharmaceutical composition of the present embodiment is 0.01 to 500 mg per unit of a dosage form as inositol derivatives; in the case of injections, the dosage is 0.02 to 250 mg per unit of a dosage form as inositol derivatives; in the case of suppositories, the dosage is 0.01 to 500 mg per unit of a dosage form as inositol derivatives; and the like. In the case of external preparations for the skin, for example, the dosage is 0.15 to 500 mg per unit of a dosage form as inositol derivatives, and it may be 0.15 to 300 mg for example, it may be 0.15 to 200 mg for example, and it may be 0.2 to 100 mg for example.

An administration interval of the pharmaceutical composition of the present embodiment may be appropriately determined according to symptoms, body weight, age, sex, and the like of a patient, a dosage form of the pharmaceutical composition, an administration method, and the like. For example, it may be once, about 2 to 3 times, or the like a day.

The pharmaceutical composition of the present embodiment can be administered to, for example, patients who have developed skin trouble such as inflammation due to contact of skin with fine particulate matter to be used for treating the skin trouble. In addition, the pharmaceutical composition of the present embodiment can also be used for promoting cell activation of skin cells in which cell proliferative activity has been reduced due to fine particulate matter. Furthermore, the pharmaceutical composition of the present embodiment can also be used for inhibiting the production of inflammation-inducing substances (such as prostaglandins) of which an amount increases due to fine particulate matter. In a preferable aspect, the pharmaceutical composition of the present embodiment is applied, as an external preparation for the skin, to an affected area in which skin trouble has developed.

Alternatively, in a case where occurrence of a high concentration of fine particulate matter is predicted, the pharmaceutical composition of the present embodiment can be prophylactically administered to patients to be used for preventing skin trouble such as inflammation caused by the fine particulate matter. In addition, the pharmaceutical composition of the present embodiment can also be used for preventing a reduction in cell proliferative activity due to the fine particulate matter. Furthermore, the pharmaceutical composition of the present embodiment can also be used for preventing an increase in the production of inflammation-inducing substances (such as prostaglandins) which is caused by fine particulate matter. In a preferable aspect, the pharmaceutical composition of the present embodiment is applied, as an external preparation for the skin, to skin predicted to come into contact with fine particulate matter.

### (Cosmetic preparation)

In one embodiment, the present invention provides a cosmetic preparation for inhibiting skin trouble caused by fine particulate matter, the composition including the above-mentioned agent for inhibiting skin trouble, and a pharmaceutically acceptable carrier.

In the cosmetic preparation of the present embodiment, the pharmaceutically acceptable carrier is not particularly limited, and carriers generally used for cosmetic preparations can be used in addition to the carriers exemplified above. For example, it is possible to use general raw materials described in the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervised by the Examination Division of the Pharmaceutical Affairs Bureau, Yakuji Nippo, Ltd., 1993); Supplement to the Japanese Cosmetic Ingredients Codex (supervised by the Examination Division of the Pharmaceutical Affairs Bureau, Yakuji Nippo, Ltd., 1993); the Comprehensive Licensing Standards of Cosmetics by Category (supervised by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The pharmaceutically acceptable carrier may be used alone or in combination of two or more kinds thereof.

In addition to the agent for inhibiting skin trouble and the pharmaceutically acceptable carrier, the cosmetic preparation of the present embodiment may contain other components. The other components are not particularly limited, and general additives for cosmetic products can be used. In addition, active ingredients other than the above-described agent for inhibiting skin trouble can be used as the other components. As additives for cosmetic products and active ingredients as the other ingredients, in addition to those exemplified above, it is possible to use general raw materials described in, for example, the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervised by the Examination Division of the Pharmaceutical Affairs Bureau, Yakuji Nippo, Ltd., 1993); Supplement to the Japanese Cosmetic Ingredients Codex (supervised by the Examination Division of the Pharmaceutical Affairs Bureau, Yakuji Nippo, Ltd., 1993); the Comprehensive Licensing Standards of Cosmetics by Category (supervised by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The other components may be used alone or in combination of two or more kinds thereof.

A form of the cosmetic preparation of the present embodiment is not particularly limited, and it can be a form generally used for a cosmetic preparation. Examples thereof include hair cosmetic preparations such as shampoos, hair conditioners, and hairdressing agents; basic cosmetic preparations such as facial cleansers, cleansing agents, skin toners, emulsions, lotions, creams, gels, sunscreens, packs, masks, and serums; makeup cosmetic preparations such as foundations, makeup primers, lipsticks, lip glosses, and blushers; body cosmetic preparations such as body cleansers, body powders, and deodorant cosmetics; and the like. These cosmetic preparations can be manufactured according to a general method. Among them, the cosmetic preparation of the present embodiment is preferably a cosmetic preparation in a form in which it is applied or attached to the skin as an external preparation for the skin. Preferable examples thereof include skin toners, emulsions, lotions, creams, gels, sunscreen agents, packs, masks, serums, foundations, makeup primers, and the like.

A dosage form of the cosmetic preparation of the present embodiment is not particularly limited, but examples thereof include emulsified types such as an oil-in-water (O/W) type, a water-in-oil (W/O) type, a W/O/W type, and an O/W/O type, emulsified polymer types, oily types, solid types, liquid types, kneaded types, stick types, volatile oil types, powder types, jelly types, gel types, paste types, cream types, sheet types, film types, mist types, spray types, multilayer types, foam types, flake types, and the like.

In the cosmetic preparation of the present embodiment, the content of the above-described agent for inhibiting skin trouble is not particularly limited, but it can be an effective amount for inhibiting skin trouble caused by the fine particulate matter. For example, a proportion of the above-described agent for inhibiting skin trouble in the cosmetic preparation of the present embodiment may be 0.01% to 50% by mass as the content of the inositol derivatives in the cosmetic preparation, and it may be 0.01% to 30% by mass for example, it may be 0.01% to 20% by mass for example, it may be 0.1% to 10% by mass for example, it may be 0.1% to 5% by mass for example, it may be 0.1% to 3% by mass for example, it may be 0.3% to 2% by mass for example, and it may be 0.6% to 1.5% by mass for example.

Regarding the content of the above-described inositol derivatives in the cosmetic preparation, in a case of using one kind of the inositol derivatives, the content refers to a content of this compound, and in a case of using two or more kinds of the inositol derivatives in combination, the content refers to the total content of these compounds.

An amount of the cosmetic preparation used of the present embodiment is not particularly limited, but it can be an effective amount for inhibiting skin trouble caused by the fine particulate matter. For example, an amount of the cosmetic preparations used of the present embodiment may be 0.15 to 500 mg per use as an amount of inositol derivatives, and it may be 0.15 to 300 mg for example, it may be 0.15 to 200 mg for example, and it may be 0.2 to 100 mg for example.

A use interval of the cosmetic preparation of the present embodiment is not particularly limited, but it can be, for example, once, about 2 to 3 times, or the like a day.

In a case where occurrence of a high concentration of fine particulate matter is predicted, the cosmetic preparation of the present embodiment can be applied to exposed skin not covered by clothing and the like (such as face and limbs) to be used for inhibiting skin trouble such as inflammation caused by the fine particulate matter. In addition, in areas or seasons where a concentration of fine particulate matter distributed is high, skin trouble such as inflammation caused by the fine particulate matter can be inhibited by using the cosmetic preparation of the present embodiment for daily skin care or makeup. Alternatively, the cosmetic preparation of the present embodiment can be applied to or pasted on skin after the skin comes into contact with the fine particulate matter to be used for reducing skin trouble such as inflammation caused by the fine particulate matter.

In addition, the cosmetic preparation of the present embodiment can also be used for inhibiting a reduction in cell proliferative activity due to the fine particulate matter. Furthermore, the cosmetic preparation of the present embodiment can also be used for inhibiting an increase in the production of inflammation-inducing substances (such as prostaglandins) which is caused by the fine particulate matter.

### [Other embodiments]

In one embodiment, the present invention provides a method for preventing or treating skin trouble (such as inflammation) caused by the fine particulate matter, the method including a step of administering, to a mammal, an inositol derivative in which a sugar (a monosaccharide or an oligosaccharide) binds to inositol.

In one embodiment, the present invention provides a method for inhibiting a reduction in cell proliferative activity caused by the fine particulate matter, the method including a step of administering, to a mammal, an inositol derivative in which a sugar (a monosaccharide or an oligosaccharide) binds to inositol.

In one embodiment, the present invention provides a method for promoting cell activation of skin cells that have come into contact with the fine particulate matter, the method including a step of administering, to a mammal, an inositol derivative in which a sugar (a monosaccharide or an oligosaccharide) binds to inositol.

In one embodiment, the present invention provides a method for inhibiting the production of an inflammation-inducing substance (such as prostaglandins) which is caused by the fine particulate matter, the method including a step of administering, to a mammal, an inositol derivative in which a sugar (a monosaccharide or an oligosaccharide) binds to inositol.

In one embodiment, the present invention provides an inositol derivative, in which a sugar (a monosaccharide or an oligosaccharide) binds to inositol, for use in preventing or treating skin trouble (such as inflammation) caused by the fine particulate matter.

In one embodiment, the present invention provides an inositol derivative, in which a sugar (a monosaccharide or an oligosaccharide) binds to inositol, for use in inhibiting a reduction in cell proliferative activity caused by the fine particulate matter.

In one embodiment, the present invention provides an inositol derivative, in which a sugar (a monosaccharide or an oligosaccharide) binds to inositol, for use in promoting cell activation of skin cells that have come into contact with the fine particulate matter.

In one embodiment, the present invention provides an inositol derivative, in which a sugar (a monosaccharide or an oligosaccharide) binds to inositol, for inhibiting the production of an inflammation-inducing substance (such as prostaglandins) which is caused by the fine particulate matter.

In one embodiment, the present invention provides use of an inositol derivative, in which a sugar (a monosaccharide or an oligosaccharide) binds to inositol, for the manufacture of an inhibitor for skin trouble (such as inflammation) caused by the fine particulate matter.

In one embodiment, the present invention provides use of an inositol derivative, in which a sugar (a monosaccharide or an oligosaccharide) binds to inositol, for the manufacture of an inhibitor against a reduction in cell proliferative activity caused by the fine particulate matter.

In one embodiment, the present invention provides use of an inositol derivative, in which a sugar (a monosaccharide or an oligosaccharide) binds to inositol, for the manufacture of a promoter for cell activation of skin cells that have come into contact with the fine particulate matter.

In one embodiment, the present invention provides use of an inositol derivative, in which a sugar (a monosaccharide or an oligosaccharide) binds to inositol, for the manufacture of an inhibitor against the production of an inflammation-inducing substance (such as prostaglandins) which is caused by the fine particulate matter.

In one embodiment, the present invention provides use of an inositol derivative, in which a sugar (a monosaccharide or an oligosaccharide) binds to inositol, for the manufacture of a composition for inhibiting skin trouble (such as inflammation) caused by the fine particulate matter.

In one embodiment, the present invention provides use of an inositol derivative, in which a sugar (a monosaccharide or an oligosaccharide) binds to inositol, for the manufacture of a composition for inhibiting a reduction in cell proliferative activity caused by the fine particulate matter.

In one embodiment, the present invention provides use of an inositol derivative, in which a sugar (a monosaccharide or an oligosaccharide) binds to inositol, for the manufacture of a composition for promoting cell activation of skin cells that have come into contact with the fine particulate matter.

In one embodiment, the present invention provides use of an inositol derivative, in which a sugar (a monosaccharide or an oligosaccharide) binds to inositol, for the manufacture of a composition for inhibiting the production of an inflammation-inducing substance (such as prostaglandins) which is caused by the fine particulate matter.

### [Examples]

Hereinafter, while the present invention will be described with reference to the following experimental examples, the present invention is not limited to the following experimental examples.

### [Experimental Example 1]

### (Production of inositol derivatives)

Myo-inositol and β-cyclodextrin were reacted in the presence of a cyclodextrin glucanotransferase to produce inositol derivatives in which glucose, or an oligosaccharide having glucose as a monosaccharide unit bound to the myo-inositol. As a result of analyzing the produced inositol derivatives by liquid chromatography-mass spectrometry (LC-MS), a percentage of molecules in which the number of glucose molecules in a glucose chain which bound to the myo-inositol was one was 12% by mass, a percentage of molecules in which the above number of glucose molecules was two was 30% by mass, a percentage of molecules in which the above number of glucose molecules was three was 9% by mass, a percentage of molecules in which the above number of glucose molecules was four was 12% by mass, and a percentage of molecules in which the above number of glucose molecules was five was 2% by mass.

### [Experimental Example 2]

### (Medium preparation and cell culture)

For fine particulate matter (PM2.5), Standard Reference Material (US registered trademark) 2783 (Air particulate on Filter Media) purchased from National Institute of Standards & Technology was used. One filter (about 500 µg of the fine particulate matter) was immersed in 1 mL of dimethyl sulfoxide (DMSO) and shaken slowly overnight to extract the fine particulate matter.

The fine particulate matter extracted as described above was added to a medium (Humedia-KG2, Kurabo Industries) so that a final concentration became 2.5 µg/cm² per culture area. Furthermore, the inositol derivatives produced in Experimental Example 1 were dissolved in purified water and added to the medium so that a final concentration became 10⁻⁵ to 10⁻³ w/v%, and thereby a medium was prepared (Examples 1 to 3). Furthermore, as a comparative example, a medium in which myo-inositol was added instead of the inositol derivatives was prepared (Comparative Examples 1 to 3). Furthermore, as control examples, media were prepared by adding, instead of the inositol derivatives, purified water in a medium to which the fine particulate matter was added, or a medium to which the fine particulate matter was not added (Control Examples 1 and 2). In these media, normal human epidermal keratinocytes (NHEK cells, Kurabo Industries) were cultured for 24 hours at 37°C in a 5% CO₂ atmosphere.

### [Experimental Example 3]

### (Effect of cell activation)

It is known that fine particulate matter damages skin cells and reduces cell proliferative activity thereof. Accordingly, an effect of cell activation by the inositol derivatives produced in Experimental Example 1 was examined using epidermal cells derived from human skin.

Cells were cultured as described in Experimental Example 2. After 24 hours from the start of culture, the cells were recovered and washed with phosphate-buffered saline. Thereafter, cell proliferative activity was measured. The measurement of cell proliferative activity was performed by a method using WST-8 (a living cell count measuring reagent SF, NACALAI TESQUE, INC.), which is a tetrazolium salt that produces water-soluble formazan. Thereafter, the number of cells was measured by a neutral red method, and a value representing cell proliferative activity was divided by a value representing the number of cells to calculate cell activation activity.

The results are shown in Table 1. In Table 1, the cell activation activity is expressed as a relative value when defining the cell activation activity of the control example (Control Example 1), in which the fine particulate matter was not added and purified water was added, as 1.00. In the control examples to which purified water was added, the cell activation activity was reduced in the group to which the fine particulate matter was added (Control Example 2) compared with that in the group to which the fine particulate matter was not added (Control Example 1). On the other hand, in the group to which the fine particulate matter and the inositol derivatives were added at the same time (Examples 1 to 3), the cell activation activity was not reduced but instead improved at any addition concentration. Meanwhile, in the group to which myo-inositol was added at the same time (Comparative Examples 1 to 3), the cell activation activity was a similar level to that in the control example in which the fine particulate matter was not added and purified water was added (Control Example 1). Based on these results, it was clarified that the inositol derivatives reduce damage to the skin cells by fine particulate matter and improve cell proliferative activity.

**[Table 1]**

| | Control Example 1 | Control Example 2 | Comparative Example 1 | Comparative Example 2 | Comparative Example 3 | Example 1 | Example 2 | Example 3 |
|---|---|---|---|---|---|---|---|---|
| Drug | Purified water | Purified water | Myo-inositol | | | Inositol derivative | | |
| Concentration (w/v%) | - | - | 10⁻⁵ | 10⁻⁴ | 10⁻³ | 10⁻⁵ | 10⁻⁴ | 10⁻³ |
| Addition of PM2.5 | PM2.5 (-) | PM2.5 (+) | PM2.5 (+) | PM2.5 (+) | PM2.5 (+) | PM2.5 (+) | PM2.5 (+) | PM2.5 (+) |
| Cell proliferative activity (relati ve value) | 1.00 | 0.84 | 0.94 | 1.00 | 1.08 | 1.08 | 1.08 | 1.14 |

### [Experimental Example 4]

### (Inhibition of production of inflammation-inducing substances)

Cells were cultured as described in Experimental Example 2. After 24 hours from the start of culture, a medium was recovered, and a concentration of the inflammation-inducing substance (prostaglandin E2) was measured by an enzyme immunoassay method (ELISA method). For the measurement, a commercially available prostaglandin E2 measurement kit (Prostaglandin E2, EIA Monoclonal Kit, Funakoshi) was used, and for a use method thereof, the attached instruction manual was followed.

The results are shown in Table 2. In Table 2, a concentration of prostaglandin E2 (PGE2) is expressed as a relative value when defining a concentration of PGE2 of the control example (Control Example 1), in which the fine particulate matter was not added and purified water was added, as 1.00. In the control examples to which purified water was added, the concentration of PGE2 increased in the group to which the fine particulate matter was added (Control Example 2) compared with the group to which the fine particulate matter was not added (Control Example 1). On the other hand, in the group to which the fine particulate matter and the inositol derivatives were added at the same time (Examples 1 to 3), the concentration of PGE2 did not increase, but evidently decreased at any addition concentration. Meanwhile, in the group to which myo-inositol was added at the same time (Comparative Examples 1 to 3), the concentration of PGE2 was a similar level or tended to slightly decrease compared with that in the control example in which the fine particulate matter was not added and purified water was added (Control Example 1). Based on these results, it was clarified that inositol derivatives inhibit the production of inflammation-inducing substances caused by the fine particulate matter.

**[Table 2]**

| | Control Example 1 | Control Example 2 | Comparative Example 1 | Comparative Example 2 | Comparative Example 3 | Example 1 | Example 2 | Example 3 |
|---|---|---|---|---|---|---|---|---|
| Drug | Purified water | Purified water | Myo-inositol | | | Inositol derivative | | |
| Concentration (w/v%) | - | - | 10⁻⁵ | 10⁻⁴ | 10⁻³ | 10⁻⁵ | 10⁻⁴ | 10⁻³ |
| Addition of PM2.5 | PM2.5 (-) | PM2.5 (+) | PM2.5 (+) | PM2.5 (+) | PM2.5 (+) | PM2.5 (+) | PM2.5 (+) | PM2.5 (+) |
| Concentration of PGE2 (relative value) | 1.00 | 1.18 | 0.97 | 0.92 | 0.86 | 0.84 | 0.83 | 0.78 |

### [Industrial Applicability]

According to the present invention, it is possible to provide an agent for inhibiting skin trouble and a composition for inhibiting skin trouble which inhibit skin trouble caused by fine particulate matter through the effects of inhibiting the production of an inflammation-inducing substance and activating cells.

## Claims

1. An agent for inhibiting skin trouble caused by fine particulate matter, the agent comprising, as an active ingredient, an inositol derivative in which a sugar binds to inositol.

2. The agent for inhibiting skin trouble according to Claim 1, wherein the sugar is a monosaccharide or an oligosaccharide.

3. The agent for inhibiting skin trouble according to Claim 2, wherein the monosaccharide is glucose.

4. The agent for inhibiting skin trouble according to Claim 2, wherein the oligosaccharide is an oligosaccharide containing glucose as a structural unit.

5. The agent for inhibiting skin trouble according to any one of Claims 1 to 4, wherein the inositol is myo-inositol.

6. The agent for inhibiting skin trouble according to any one of Claims 1 to 5, which inhibits the production of an inflammation-inducing substance.

7. The agent for inhibiting skin trouble according to any one of Claims 1 to 6, which promotes cell activation.

8. A composition for inhibiting skin trouble caused by fine particulate matter, the composition comprising:
the agent for inhibiting skin trouble according to any one of Claims 1 to 7; and
a pharmaceutically acceptable carrier.

9. The composition for inhibiting skin trouble according to Claim 8, wherein the content of the inositol derivative is 0.01 % to 50% by mass.

10. The composition for inhibiting skin trouble according to Claim 8 or 9, which is an external preparation for skin.

11. The composition for inhibiting skin trouble according to any one of Claims 8 to 10, which is a cosmetic preparation.
